Europäisches Patentamt

European Patent Office

Office européen des brevets

(11) Publication number : **0 428 241 B1**

(12) **EUROPEAN PATENT SPECIFICATION**

(45) Date of publication of patent specification :
12.07.95 Bulletin 95/28

(51) Int. Cl.⁶ : **G01N 33/10, G01N 7/00**

(21) Application number : 90301481.9

(22) Date of filing : 13.02.90

(54) Method and device for control of dough mixing.

(30) Priority : **13.11.89 JP 292363/89**

(43) Date of publication of application :
**22.05.91 Bulletin 91/21**

(45) Publication of the grant of the patent :
**12.07.95 Bulletin 95/28**

(84) Designated Contracting States :
**CH DE ES FR GB IT LI NL**

(56) References cited :
**US-A- 4 766 766**
**US-A- 4 838 081**
**SOVIET PATENT ABSTRACTS, Chemical Patents Index, week 9031, Derwent Publications LTD., London, D11 (GB)/**
**SOVIET INVENTIONS ILLUSTRATED, D section, week X7, 24 March 1976, Derwent Publications LTD., London (GB)/**
**SOVIET INVENTIONS ILLUSTRATED, Chemical: I section, week W24, 22 July 1975, Derwent Publications LTD., London, D11 (GB)/**

(56) References cited :
**SOVIET INVENTIONS ILLUSTRATED, section III, Mechanical & General, March 1971, Derwent Publications LTD., London (GB)/**
**JAPANESE UTILITY MODEL PUBLICATION, no. SHO 62-33566/**

(73) Proprietor : **SYSTEM SOUGOU KAIHATSU CO., LTD.**
**8-17, Hongo 4-chome,**
**Bunkyo-ku**
**Tokyo (JP)**

(72) Inventor : **Inabayashi, Shoji**
**1153-1, Ageo-mura**
**Ageo-shi, Saitama-ken (JP)**
Inventor : **Nakae, Toshiaki**
**14-26-411, Shimomae 1-chome**
**Toda-shi, Saitama-ken (JP)**
Inventor : **Asakawa, Tetsuji**
**576-36, Minami-hirano**
**Iwatsuki-shi, Saitama-ken (JP)**

(74) Representative : **Watkins, David et al**
**Urquhart-Dykes & Lord**
**91 Wimpole Street**
**London W1M 8AH (GB)**

## Description

This invention relates to a method for control of a dough mixing process, capable of determining the completion of mixing dough in production of bread and to a device used for practicing this method.

There has been conventionally known a method for control of dough mixing, in which a mixing process for making up dough required for producing bread is finished at the time that a prescribed time elapses after the peak value of viscoelasticity of the dough is found by detecting the peak of load power successively consumed by a motor incorporated in a dough mixer, as proposed by Japanese Utility Model Publication No. SHO 62-33566, for example.

As well, there has been widely employed a method in which the dough mixing process is controlled by use of a timer for indiscriminatingly deciding the completion of dough mixing.

In the meantime, since load power consumed by a mixing motor incorporated in a dough mixer is varied in respective processes of thrusting, stretching and punching dough in the actual dough mixing, the load power consumed by the mixing motor changes pulsationally.

The former method for controlling the dough mixing performs an arithmetic operation for eliminating pulsation components in electric power applied to the mixing motor. However, such an arithmetic operation fundamentally gives rise to time delay for signal transmission. Therefore, it is difficult to precisely detect the true peak of viscoelasticity of the dough being mixed on the basis of the load power consumed by the mixing motor in mixing the dough.

The change of the load power actually consumed by the mixing motor depends on not only the viscoelasticity of the dough but also the movement and change in temperature of the dough being mixed in the mixer. As a result, local peaks of load power frequently take place in one mixing process. Hence, even though the peak of the load power consumed by the mixing motor can be precisely detected, it is difficult to ascertain the only peak of viscoelasticity of the dough in mixing.

There has been another method of control for making bread, in which a mixing process is finished in a slight stirring state (under-mixing state) before the viscoelasticity of the dough being mixed reaches the maximum. In this method it is not practical to detect the aforementioned peak of viscoelasticity of the mixed dough.

The aforesaid method employing a timer for determining the completion of a dough mixing process has been actually used as a practicable method in many bakeries. However, the time required for assuring a desirable viscoelasticity of the dough changes subtly with various conditions such as mixing temperature, water content in the dough and so on. Thus, the time at which the dough mixing is adequately finished can in no way be determined with precise by such a timer.

This invention is made to eliminate the drawbacks suffered by the conventional methods as noted above and aims at providing a method and device for control of dough mixing, capable of precisely determining the completion of the dough mixing on the basis of the electric power quantity consumed with dough mixing.

Another object of this invention is to provide a method and device capable of automatically calculating the electric power quantity required for obtaining suitable dough relative to the weight of the dough.

To attain the objects described above according to this invention, there is provided a method for control of dough mixing, which comprises detecting load power consumed by a mixing motor for driving a mixer in which reference dough is placed, integrating the detected load power during mixing of the reference dough until optimum reference dough is obtained so as to determine a standard power quantity value relative to ingredient type and weight of the dough, mixing objective dough with said mixer while detecting load power consumed by the mixing motor of the mixer, integrating the detected load power during mixing of the objective dough to obtain actual power quantity, and deciding completion of the mixing of said objective dough when the actual power quantity reaches said standard power quantity value.

By previously determining the standard value relative to the weight of reference dough to obtain a proportional relation between itself and the weight of the reference dough, the standard value determined for objective dough to be mixed can be readily obtained from the proportional relation therebetween.

A device for practising the aforementioned method for control of dough mixing according to this invention comprises a dough mixer driven by a mixing motor, which comprises a data input unit for inputting data representing ingredient type and weight of dough to be mixed and electric power quantity required for mixing the dough, a standard value determining unit for determining standard power quantity value on the basis of the data inputted in said data input unit, a power detection unit for detecting load power consumed by said mixing motor, an arithmetic unit for calculating electric power quantity value from the load power detected by said power detection unit, and a decision unit for deciding completion of the dough mixing when the electric power quantity calculated by said arithmetic unit reaches the standard power quantity value determined by said standard value determining unit.

In the aforementioned method according to the invention, the electric power quantity serving as the standard value for deciding the completion of the dough mixing is previously programmed on the basis of the data obtained by mixing the reference dough relative to the conditions such as the ingredient type and weight of the reference dough. Then, the mixing of objective dough to be mixed are monitored and conformed while detecting and integrating load power consumed by the mixing motor to calculate electric power quantity consumed with mixing, so as to predetermine the standard value in terms of electric power. Otherwise, in a case that the standard value relative to the type of dough has been already predetermined, a specific standard value can be automatically calculated merely by inputting the weight of ingredients such as flour to be used.

In mixing objective dough, the load power consumed by the mixing motor is successively detected while being integrated to obtain electric power quantity so that the completion of the dough mixing can be decided when the integrated power quantity reaches the predetermined reference value.

In the device according to the invention, at the outset, the data such as the type and weight of the reference dough under test and electric power quantity consumed with mixing are given to the data input device so as to determine the standard value by the standard value determining unit on the basis of the data thus inputted. During the mixing operation performed by driving the mixing motor, the load power being dissipated by the mixing motor is successively detected by the power detection unit. Simultaneously, the power quantity consumed by the mixing motor is calculated from the detected load power in the arithmetic unit, so that the completion of the dough mixing can be decided by the decision unit when the electric power quantity thus calculated reaches the standard value determined by the standard value determining unit.

The other objects and features of the present invention will now be explained in detail with reference to the accompanying drawings, wherein:-

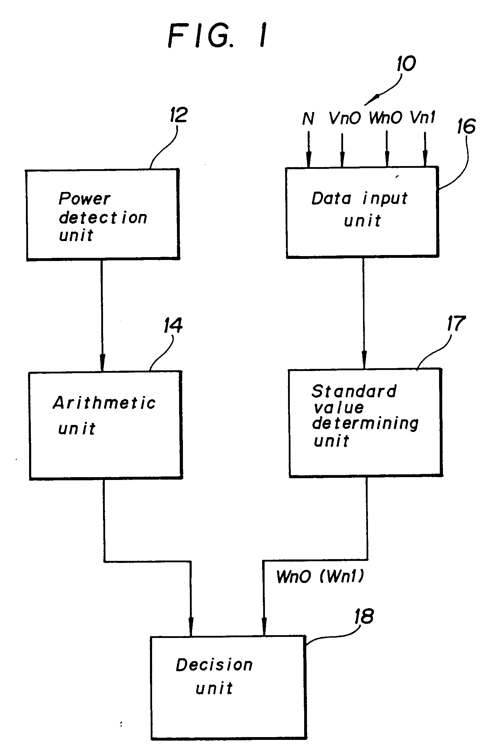

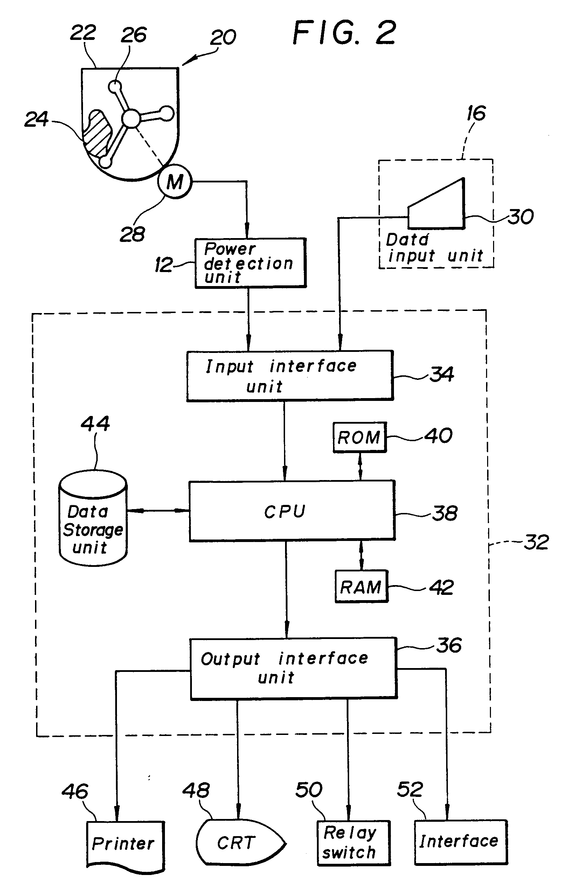

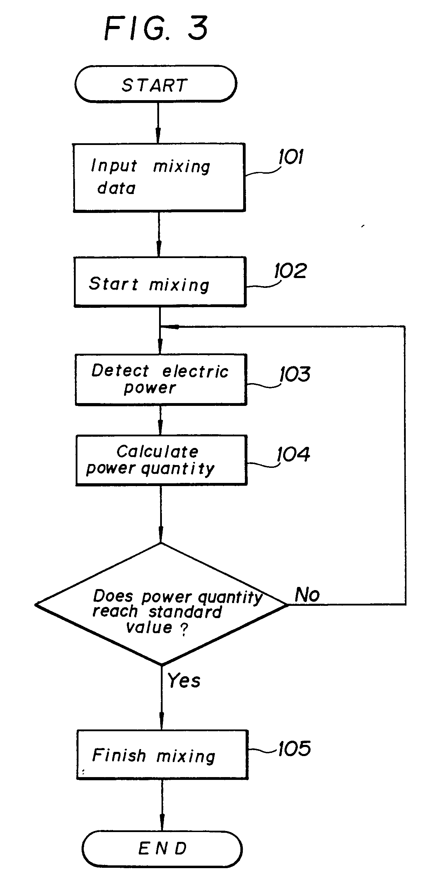

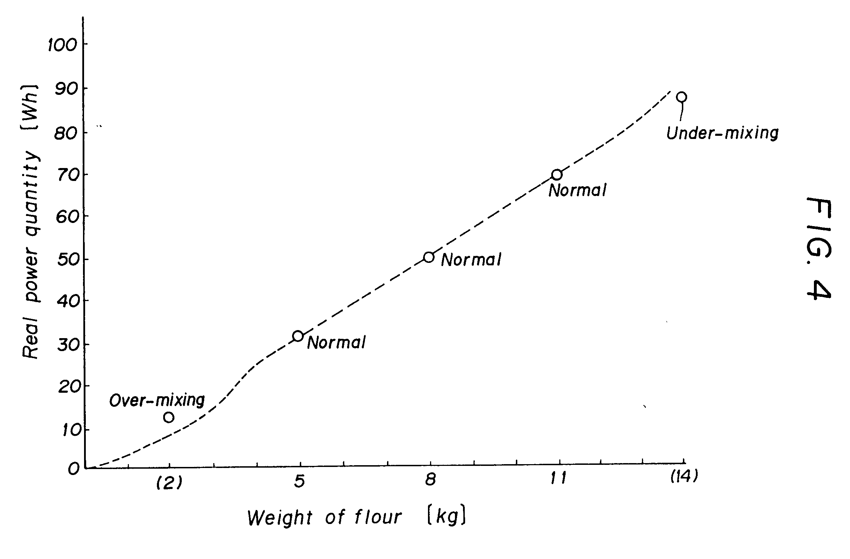

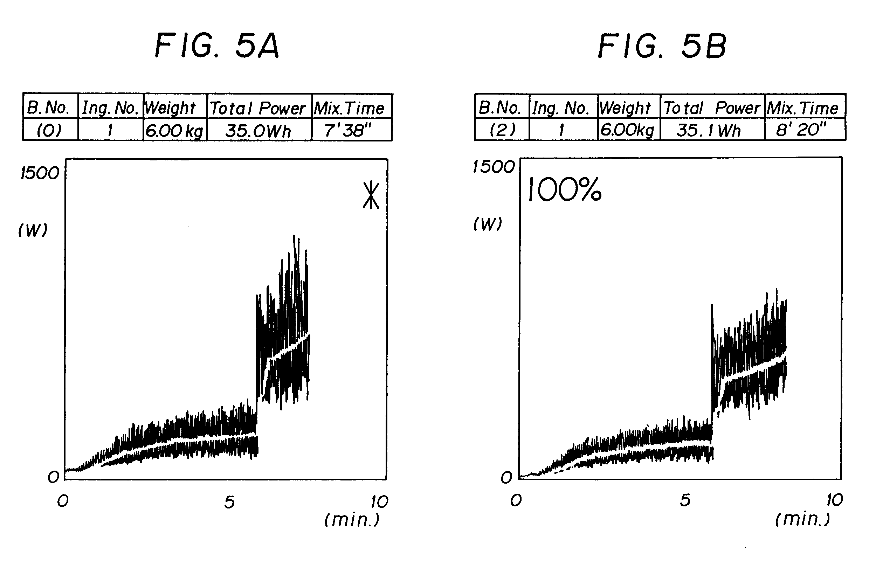

Figure 1 is a conceptional block diagram showing one embodiment of the device for the control of dough mixing according to this invention; Figure 2 is a block diagram showing the device to be used for working the method for the control of dough mixing according to this invention; Figure 3 is a flow chart illustrating the procedure of dough mixing according to the method of this invention; Figure 4 is a graph showing the relation between the power quantity and the weight of dough; and Figures 5A to 5C are explanatory diagrams respectively illustrating an image pattern displayed on a monitor screen as the result of dough mixing according to the invention.

This invention relates to a method and device for the control of dough mixing, capable of precisely deciding the completion of the dough mixing so as to obtain the optimum dough for bread. First of all, the principle for practicing the method according to this invention for adequately deciding the completion of the dough mixing will be described hereinafter.

The inventors of this invention have taken note of the universality present in electric power quantity consumed for dough mixing to evaluate the dough being mixed while precisely measuring change of the load power for driving a mixing motor. The experiments for analyzing the results obtained from the actual dough mixing have been repeatedly made. The following Table 1 and Table 2 represent the results of the experiments conducted by the inventors by way of example.

## TABLE 1

```
Type of mixer    : Horizontal-tape mixer made by Fujisawa
                   Seisakusho K.K. of Japan; Model No: FH-2
Rated capacity   : 50 kg
Rated voltage    : AC 200 V
Rated power      : 5.5 kW at high speed
                   2.75 kW at low speed
Type of dough    : Loaf of white bread (cont. 25kg flour)
Mixing method    : Straight dough mixing method
```

| Batch | Power Quantity (kWh) | Mixing Time | Mixing-up Temp.(°C) | Condition of dough |
|---|---|---|---|---|
| (1) Standard | 0.553 | 17min 1sec | 28.0 | Normal |
| (2) Identical with (1) | 0.555 | 16min 45sec | 27.6 | Normal |
| (3) +3% Water content | 0.557 | 17min 2sec | 27.4 | Normal |
| (4) -3% Water content | 0.555 | 16min 41sec | 28.0 | Normal |
| (5) Water charged at 27°C (increase by 10°C) | 0.660 | 19min 44sec | 27.0 | Forced cooling Mixing started en route |

In all the batches (1) to (4), adequate dough could be obtained by finishing the dough mixing when the power quantity consumed by the mixer reaches the fixed value (the last figures in the item "Power Quantity" of Table 1 are not significant because of an error in measurement).

Specifically even in the case of the so-called "water absorption" suggesting variations in the amount of water content in the dough and the mixing temperature, no abnormality was recognized. The batch (5) was tested under an extremely abnormal condition in temperature. The mixer was stopped in the course of mixing the dough at a temperature of 27°C. In this case, it was confirmed that the dough was insufficiently mixed. This fact implies that, if the mixing temperature exceeds a predetermined limit in temperature, normal dough cannot be obtained even under the same condition of power quantity.

## TABLE 2

Type of mixer  : Vertical-tape mixer made by KEMPER
                   Model No: SP15L
Rated capacity : 15 kg
Type of dough  : French bread
Mixing method  : Straight dough mixing method
Mixing time    : 7 min. at low speed.
                   The time at high speed is noted below.

| Batch | Power Quantity (Wh) | High-speed Mixing Time | Mixing-up Temp.($^\circ$C) | Condition of dough |
|---|---|---|---|---|
| (1) Standard (8kg flour) | 91.8 [49.5] | 1min 37sec | 24.0 | Normal |
| (2) Identical with (1) (8kg flour) | 91.8 [49.5] | 1min 37sec | 24.0 | Normal |
| (3) Using long-stored flour (8kg flour) | 91.0 [48.5] | 1min 40sec | 23.8 | Normal |
| (4) Identical with (1) (5kg flour) | 72.9 [31.5] | 1min 31sec | 24.3 | Normal |
| (5) Identical with (1) (11kg flour) | 110.2 [68.8] | 1min 28sec | 23.7 | Normal |

In the above table, the numerical values in the brackets in "Power Quantity" respectively mean the real power quantity obtained by subtracting the power quantity consumed by driving the mixing motor under loadless from the power quantity consumed when mixing the dough. The batches (1) to (3) in Table 2 proves that it is successful for obtaining adequate dough to finish the dough mixing at the time when the real power quantity substantially reaches the specific power value. Specifically, it is proved from the batch (3) that the predetermined standard value is applicable even to the dough mixing using the long-stored flour with water content different from that in the flour of the reference dough. The above Table 2 still clarifies that, in the case of increasing or decreasing the amount of dough to be mixed as in the batch (4) or (5), the normal dough can be properly obtained by finishing the dough mixing when the power quantity successively detected during mixing reaches the predetermined standard value specified in the batch (1).

The following conclusions could be drawn from the aforementioned experimental results.

i) The electric power quantity required for mixing the dough of the same ingredients and the same weight is constant under normal mixing conditions. The term "normal conditions" implies that variations in temperature and water content in flour are within allowable range. The allowable range in the experiments which were actually conducted by the inventors of this invention was set to $\pm1^\circ$C in temperature and $\pm3\%$ in coefficient of water absorption relative to the respective standard values as the desired values. As termed herein, "weight of dough" is a substantially synonym for "weight of flour" in view of ingredients.

ii) The real power quantity value V1 required for mixing the dough of the weight value W1 within a fixed extent in capacity of the mixer is equal to that obtained by multiplying the standard power quantity value V0 required for mixing the dough of the weight value W0 composed of the same ingredients by the ratio W1/W0 in weight. The power quantity actually measured includes, in general, no-loading power quantity represented by multilying the electric power consumed when the mixer motor is raced under loadless by the operating time of the motor. Thus, the power quantity values V0 and V1 as noted above can be rewritten by values obtained by subtracting the respective no-loading power quantity values V1b and V0b from the actually measured power quantity value V1a and V0a. This can be formulated as follows.

$$V1a - V1b = (V0a - V0b) \times W1/W0 \quad (1)$$

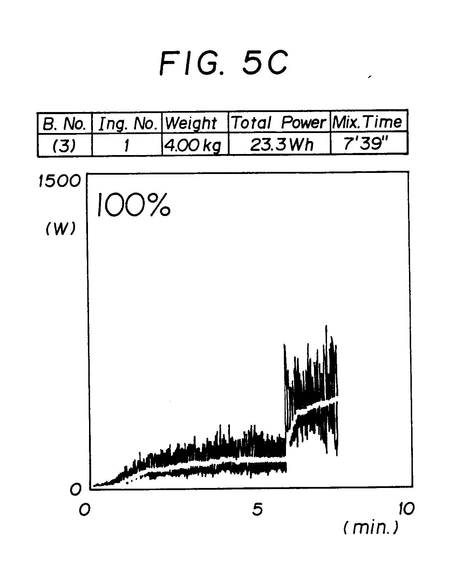

The term "fixed extent" in capacity of the mixer implies the extent in which the weight of the dough is kept proportional to the power quantity required for mixing the dough. As is apparent from Table 2, the conditions in the extent of the weight from 5 kg to 11 kg can be deemed to be in proportion to that of the standard weight of 8 kg. The graph illustrated in Figure 5 represents the relation between the weight of the dough to be mixed and the power quantity for mixing the dough.

Next, the device for embodying the principle mentioned above according to this invention will be described with reference to Figures 1 and 2.

The device 10 for the control of dough mixing according to the invention comprises a power detection unit 12, an arithmetic unit 14, a data input unit 16, a standard value determining unit 17 and a decision unit 18 as illustrated in Figure 1.

The ingredients constituting the desired dough 24 is first placed in a mixing bowl 22 of a mixer 20 in which the dough is kneaded by rotating an agitator 26 by use of a mixing motor (M) 28. The motor 28 is supplied with electric power which is monitored by the power detection unit 12. That is, load power consumed by actuating the motor 28 is successively detected by the power detection unit 12. The load power thus detected is momentarily integrated by the arithmetic unit 14 to calculate electric power quantity by means of a processing circuit designed in consideration of power factor which is obtained from both the voltage signal and current signal given to the fixer.

The data input unit 16 may be provided with a keyboard 30 for manually inputting processing instructions or mixing data. The mixing data to be inputted to the data input unit 16 include data for predetermining the standard values relative to several kinds of specific ingredient types and weight values of the reference dough and data for specifying one of the predetermined ingredient types and setting the weight value of the objective dough to be fixed. The former data as to the standard value are inputted with the keyboard 30 to set the ingredient type $\underline{N}$ and the weight value Wn0 of the reference dough under test, so that the power quantity, i.e. standard value Vn0, required for mixing the reference dough is integrated and consequently registered. The latter data to be inputted are of the ingredient type $\underline{N}$ and weight value Wn1 of the objective dough to be actually mixed.

The aforenoted arithmetic unit 14, standard value determining unit 17 and decision unit 18 can be constructed by a processing system 32 composed of microcomputers. The processing system 32 comprises an input interface circuit 34, an output interface circuit 36, a central processing unit (CPU) 38, a read-only memory (ROM) 40, a random-access memory (RAM) 42, and a data storage unit 44.

The input interface circuit 34 serves to convert input signals into the prescribed signal forms and is provided with analog input terminal means for receiving electric power signals given in analog representation by the power detection unit 12 and digital input terminal means corresponding to keys on the keyboard 30.

The CPU 38 plays a pivotal role of the device 10 in conformity to a program written in the ROM 40, namely functions to read data, perform various operations, store the data, compare the data for decision, and deliver output signals. To put it concretely, it has a function of integrating the electric power detected by the power detection unit 12 to obtain power quantity consumed for mixing the dough and comparing the power quantity thus obtained with the standard value corresponding to the mixing data inputted from the keyboard 30.

The data storage unit 44 is constructed by integrated circuit memories, magnetic bubble memories or the like and functions to store mixing data detected sequentially and various standard data for decision. The data stored in the data storage unit 44 can be held by use of a backup battery.

The output interface circuit 36 functions to output signals for displaying the mixing data actually measured and decision results under the control of the CPU 38. The signal to stop the mixer outputted via the output interface unit 36 is delivered to an acoustic indicator, alarm lamp, and/or external computer system. To the interface circuit 36, there may be connected a printer 46, cathode ray tube (CRT) 48, relay switch 50 for breaking the circuit to the mixer, and interface 52 for communication with external computers.

Still, the power detection unit 12 and arithmetic unit 14 can of course be incorporated so as to constitute the so-called wattmeter.

The method and device according to this invention have two fundamental functions of registering the standard values for the control of dough mixing and controlling the dough mixing process by using the registered standard values every mixing batch.

The registration of the standard values is accomplished as follows. First, the ingredient type $\underline{N}$ and weight value Wn0 corresponding to the type and weight of the reference dough of which standard values are intended to be registered are inputted with the keyboard 30 of the device 10. Next, dough mixing is actually carried out under the control of a timer or the like as generally employed in a common dough mixer, and then, the dough obtained as the result of mixing is evaluated in its mixed state so as to find power quantity data on which adequately mixed dough is obtained. The power quantity data thus found are registered as the standard value Vn0 serving as criterion in mixing the objective dough. In this case, the time for mixing the dough may be somewhat shortened in respect of that in which adequately mixed dough is obtained, so that the time of mixing the dough can be arbitrarily extended while observing the mixed state of the dough under mixing until the dough comes to be the optimum. Instead of the timer as noted above, there may be inputted tentative power quantity value to the data input unit 16 so that the mixing condition can be progressively observed consequently to find the standard value Vn0.

Next, the method according to this invention for the control of dough mixing will be described in detail with reference to the flow chart shown in Figure 3.

First of all, the data such as the type $\underline{N}$ and weight Wn0 of the reference dough, the standard power quantity value Vn0, and the weight Wn1 of the objective dough to be mixed actually are manually inputted in advance from the keyboard 30 (Step 101).

As described above in connection with the step 101, the present invention has a characteristic feature in that, in the case that the batch data such as the standard weight value Wn0 of the reference dough having the same type $\underline{N}$ has been registered, the power quantity value Vn1 for determining the completion of the dough mixing is automatically calculated merely by inputting the type $\underline{N}$ and weight value Wn1 to the device 10 by use of the keyboard 30.

The term "type of dough" used herein is represented by a combination of the following factors.

1. Kind of bread, such as French bread and loaf of white bread.
2. Percentage, i.e. blending ratio of ingredients such as flour and water.
3. Mixing conditions such as a straight dough mixing method and sponge dough mixing method.

At this time, the standard power quantity value Vn0 or Vn1 for deciding the completion of the dough mixing is determined. Then, the ingredients for the desired objective dough 24 are placed in the mixer 20 and mixed by rotating the agitator 26 (Step 102). Simultaneously, the load power consumed by actuating the mixing motor 28 to rotate the agitator 26 is detected (Step 103), while successively integrating the load power thus detected to obtain the actual power quantity and subtracting no-loading power quantity measured in advance from the actual power quantity to obtain the real power quantity (Step 104). At last, when the real power quantity thus obtained reaches the predetermined standard power quantity value, a decision instruction to stop the mixer is issued Step 105).

To be more specific, an operator first inputs the standard values Vn0 and the weight Wn0 corresponding to objective dough of type $\underline{N}$ to be mixed in preparation for an actual mixing operation. At this time, the operator freely chooses, from among some conditions programmed in advance, characteristic condition prescribed so as to enable the objective dough to be mixed adequately. As a result, the standard power quantity value Vn0 is automatically determined.

In a case that another dough of the type $\underline{N}$ whose standard batch data Wn0, Vn0 have been previously is newly subjected to mixing, informations of the type $\underline{N}$ and weight Wn1 of the objective dough to be mixed are inputted to the device 10 by use of the keyboard 30, so that the standard power quantity value Vn0 which the mixing motor just consumes for producing the optimum dough is automatically calculated. This can be formulated by use of Equation (1) described above, as follows:

$$Vn1 \; = \; Vn0 \; x \; (Wn1/Wn0) \quad (2)$$

As is apparent from the above, according to the method of the invention, even if the conditions in temperature and water content in the dough are varied, or dough being mixed twines round the agitator of the vertical-type mixer resulting in insufficient mixing, the mixing time correspondingly changes so that the mixer is stopped when the power quantity actually consumed for mixing the dough reaches the predetermined standard power quantity value. consequently, the desirable dough having fixed viscoelasticity can be readily produced. Still, fluctuation in load on the mixing motor may be displayed on the CRT 48 or other display means together with the predetermined standard batch data. Furthermore, the mixer 20 may be automatically stopped when issuing the decision of the completion of mixing, and simultaneously, the acoustic indicator, lamp, and/or buzzer may be automatically actuated to give a warning to the operator. It is also possible to transfer the output data from the device to an external computer system and/or print out the data on the printer 46 in accordance with the instruction signals outputted from the interface unit 52 for communication.

Some concrete examples of the method for the control of dough mixing according to this invention will be elucidated in accordance with the following experimental results.

[Experimental data]

| | |
|---|---|
| Mixer used for test : | KEMPER-SP15L |
| Rated capacity : | 15 kg |
| Type of dough for : | French bread |

```
Ingredients (% on flour basis):
                1. Flour     6000g (100%)
                2. Salt       120g   (2%)
                3. Yeast       60g   (1%)
                4. Improver    12g  (0.2%)
                5. Malt        12g  (0.2%)
                6. Water     4080 cm³ (68%)
```

As the result of mixing the dough having the aforenoted ingredients prepared for reference dough for 6 minutes at low speed and otherwise 1 minute 40 seconds at high speed, the mixed dough in the normal state could be obtained. Then, power quantity of 35.0 Wh consumed by the mixing was registered as standard value. Thereafter, the experiments were conducted in the manner of the following examples, consequently to confirm the effect of the present invention.

1. Mixing of dough identical in ingredient and weight with the reference dough used as the criterion.

2. Mixing of dough identical in ingredient and weight with the reference dough, but including flour made by a different flour maker.

3. Mixing of dough having the identical ingredient and weight two thirds that of the reference dough.

The experimental results are shown in Table 3 below.

### TABLE 3

| Batch | Weight (kg) | Mixing Time | Power Quantity | Mixing-up Temp. (°C) | Condition of dough |
|-------|-------------|-------------|----------------|----------------------|--------------------|
| (0) | 6 | 1m 40s | 35.0Wh | 26.7 | Standard |
| (1) | 6 | 1m 44s | 35.2Wh | 27.0 | Equality |
| (2) | 6 | 2m 20s | 35.1Wh | 26.8 | Equality |
| (3) | 4 | 1m 39s | 23.3Wh | 26.0 | Equality |

In Table 3, the time data in the case of high speed mixing are shown. Low speed mixing on the respective mixing batches were complete in 6.0 minutes at all times. The scattering in the measured values of the batches (0), (1) and (2) in "Power Quantity" of Table 3 is caused by error in accuracy arising from the decision which was made every one second for finishing the dough mixing.

As is evident from the experimental results shown in Table 3, irrespective of variations in mixing-up temperature and water content in flour (dependent on flour lots), it has been confirmed that the decision for finishing the dough mixing which is performed using the standard values determined in the standard batches experimentally carried but is successful for adequately mixing the dough for bread. Moreover, the experimental result on the batch (3) shown in Table 3 proves that compensation for calculation of the standard value relative to decrease in weight of dough was availably effected.

Figures 5A to 5C show hard copies produced respectively from an image pattern displayed on a monitor screen such as a CRT 48 of the device according to this invention. The displayed image patterns illustrated reveal that the dough mixing in the batches (2) and (3) in Table 3 were finished true to the standard mixing shown in Figure 5A (at 100% of the standard value in power quantity). The standard power quantity value in the batch (3) was corrected in accordance with the ratio in weight of the objective dough to the reference dough under test, namely the full scale (100%) of the standard power quantity value in the batch (3) is adjusted to 23.3Wh. Though a vertical type spiral mixer was used in these experiments noted above, the method of this invention can of course be applied widely to every type of mixer such as a horizontal type mixer of large capacity and a vertical type mixer of small capacity.

As described above in detail, according to this invention, since the completion of the dough mixing process is decided by the electric power quantity required for mixing dough, the mixing is adequately finished with a high degree of precise, thereby to stably produce the dough of high quality. Besides, since the power quantity required for mixing can be automatically calculated from weight of arbitrary dough, the operational efficiency for operating the device for practicing the method of the invention can be notably improved.

Thus, the device for the control of dough mixing according to this invention can be simply constructed and offer a function of precisely finishing the dough mixing on the basis of the electric power quantity required for adequately mixing the dough.

## Claims

1. A method for control of dough mixing, which comprises detecting load power consumed by a mixing motor (28) for driving a mixer (20) in which reference dough is placed, integrating the detected load power during mixing of the reference dough until optimum reference dough is obtained so as to determine a standard power quantity value relative to ingredient type and weight of the dough, mixing objective dough with said mixer while detecting load power consumed by the mixing motor (28) of the mixer (20), integrating the detected load power during mixing of the objective dough to obtain actual power quantity, and deciding completion of the mixing of said objective dough when the actual power quantity reaches said standard power quantity value.

2. A method according to Claim 1, wherein the standard power quantity value relative to the weight of the objective dough to be mixed is calculated on the basis of a proportional relation between the standard power quantity value and the weight of the objective dough.

3. A device for control of dough mixing in a dough mixer (20) driven by a mixing motor (28), which comprises a data input unit (10) for inputting data representing ingredient type and weight of dough to be mixed and electric power quantity required for mixing the dough, a standard value determining unit (17) for determining standard power quantity value on the basis of the data inputted in said data input unit (10), a power detection unit (12) for detecting load power consumed by said mixing motor (28), an arithmetic unit (14) for calculating electric power quantity value from the load power detected by said power detection unit (12), and a decision unit (18) for deciding completion of the dough mixing when the electric power quantity calculated by said arithmetic unit (14) reaches the standard power quantity value determined by said standard value determining unit (17).

## Patentansprüche

1. Verfahren zum Steuern des Mischens von Teig, welches die Erfassung der Lastenergie umfaßt, die von einem Mischmotor (28) für den Antrieb eines Mischers (20) verbraucht wird, in welchem ein Referenzteig angeordnet wird, ein Integrieren der erfaßten Lastenergie während des Mischens des Referenzteiges, bis ein optimaler Referenzteig erhalten wird, um einen Mengenwert einer Standardleistung relativ zu dem Bestandteiltyp und dem Gewicht des Teigs zu bestimmen, einem Mischen des betroffenen Teigs mit dem Mischer, während die durch den Mischmotor (78) des Mischers (20) verbrauchte Lastenergie erfaßt wird, einem Integrieren der erfaßten Lastenergie während des Mischens des betroffenen Teigs, um die tatsächliche Leistungsmenge zu erhalten, und einer Entscheidung über die Beendigung des Mischens des betroffenen Teigs, wenn die tatsächliche Energiemenge den Mengenwert der Standardleistung erreicht.

2. Verfahren nach Anspruch 1, bei welchem der Mengenwert der Standardleistung relativ zu dem Gewicht des betroffenen, zu mischenden Teigs auf der Basis einer Proportionalbeziehung zwischen dem Mengenwert der Standardleistung und dem Gewicht des betroffenen Teigs berechnet wird.

3. Vorrichtung zum Steuern des Mischens eines Teigs in einem Teigmischer (20), der durch einen Mischmotor (28) angetrieben wird, bestehend aus einer Dateneingabeeinheit (10) für eine Eingabe von Daten, welche den Bestandteiltyp und das Gewicht des zu mischenden Teigs angeben sowie die für das Mischen des Teigs benötigte elektrische Energiemenge, eine Standardwert-Bestimmungseinheit (17) für eine Bestimmung des Mengenwerts der Standardleistung auf der Basis der in die Dateneingabeeinheit (10) eingegebenen Daten, einer Leistungserfassungseinheit (12) zum Erfassen der durch den Mischmotor (28) verbrauchten Lastenergie, einer Rechnereinheit (14) für ein Berechnen des Mengenwertes der elektrischen Leistung aus der Lastenergie, die durch die Lasterfassungseinheit (12) erfaßt wird, und einer Entscheidungseinheit (18) für die Entscheidung einer Beendigung des Teigmischens, wenn die durch die Rechnereinheit (14) berechnete Menge der elektrischen Leistung den Mengenwert der Standardleistung erreicht, der durch die Bestimmungseinheit (17) des Standardwertes bestimmt wurde.

## Revendications

1. Procédé de commande de mélange de pâte à pain, qui comprend la détection de l'énergie consommée par un moteur (28) pour l'entraînement d'un malaxeur (20) dans lequel est placée de la pâte de référence, l'intégration de l'énergie détectée pendant le malaxage de la pâte de reférence jusqu'à l'obtention d'une pâte de référence optimale, de manière à déterminer une valeur type de quantité d'énergie par rapport au type d'ingrédients et au poids de la pâte, le malaxage de pâte réelle par ledit malaxeur tout en détectant l'énergie consommée par le moteur (28) du malaxeur (20), l'intégration de l'énergie détectée pendant le malaxage de la pâte réelle pour obtenir la quantité d'énergie réelle, et la décision que le malaxage de ladite pâte réelle est terminé lorsque la quantité d'énergie réelle atteint ladite valeur type de quantité d'énergie.

2. Procédé selon la revendication 1, dans lequel la valeur type de quantité d'énergie par rapport au poids de la pâte réelle à malaxer est calculée sur la base d'une relation proportionnelle entre la valeur type de quantité d'énergie et le poids de la pâte réelle.

3. Dispositif pour la commande du malaxage dans un malaxeur de pâte à pain (20) entraîné par un moteur (28), qui comprend une unité d'entrée de données (10) pour entrer des données représentant le type d'ingrédients et le poids de la pâte à malaxer et la quantité d'énergie électrique nécessaire au malaxage de la pâte, une unité de détermination de valeur type (17) pour déterminer la valeur type de quantité d'énergie sur la base des données entrées dans ladite unité d'entrée de données (10), une unité de détection d'énergie (12) pour détecter l'énergie consommée par ledit moteur (28), une unité arithmétique (14) pour calculer la valeur de la quantité d'énergie électrique à partir de l'énergie détectée par ladite unité de détection d'énergie (12), et une unité de décision (18) pour décider que le malaxage de la pâte est terminé lorsque la quantité d'énergie électrique, calculée par ladite unité arithmétique (14) atteint la valeur type de quantité d'énergie déterminée par ladite unité de détermination de valeur type (17).

# FIG. 1

FIG. 2

# FIG. 3

START

Input mixing data — 101

Start mixing — 102

Detect electric power — 103

Calculate power quantity — 104

Does power quantity reach standard value ? — No

Yes

Finish mixing — 105

END

FIG. 4

## FIG. 5A

| B. No. | Ing. No. | Weight | Total Power | Mix. Time |
|--------|----------|--------|-------------|-----------|
| (0) | 1 | 6.00 kg | 35.0 Wh | 7' 38" |

## FIG. 5B

| B. No. | Ing. No. | Weight | Total Power | Mix. Time |
|--------|----------|--------|-------------|-----------|
| (2) | 1 | 6.00 kg | 35.1 Wh | 8' 20" |

EP 0 428 241 B1

# FIG. 5C

| B. No. | Ing. No. | Weight | Total Power | Mix. Time |
|--------|----------|--------|-------------|-----------|
| (3) | 1 | 4.00 kg | 23.3 Wh | 7'39" |